# DEMANDE DE BREVET EUROPEEN

(11) **EP 4 574 991 A1**
(43) Date de publication de la demande: **25.06.2025**
(21) Numéro de dépôt: 23217648.7
(22) Date de dépôt: 18.12.2023
(51) Int. Cl.: C12Q 1/04, C12Q 1/22, G01N 33/50, G01N 33/569

(54) **MÉTHODE POUR DÉTECTER DES MICROORGANISMES VIABLES POTENTIELLEMENT PRÉSENTS DANS UN ÉCHANTILLON DE PRODUITS CELLULAIRES**

(71) Demandeur: bioMérieux, 69280 MARCY-L'ETOILE (FR)
(72) Inventeur: DE LA FOATA, Corinne, 69480 Pommiers (FR); GALLET-GORIUS, Emmanuelle, 69700 Echalas (FR); PALAZUELO, Marc, 69290 Grezieu-la-Varenne (FR); RAYMOND, Jean-Claude, 69690 Bessenay (FR)
(74) Mandataire: bioMérieux PI Groupement mandataires

(57) **Abrégé**

La présente invention concerne un procédé d'isolement de microorganismes viables potentiellement présents dans un échantillon comprenant 10⁶ à 10⁸ cellules immunitaires ou cellules progénitrices de ces dernières, comprenant les étapes suivantes :
- Mettre en contact l'échantillon avec
• une composition lysante comprenant
∘ un tampon de lyse comprenant un détergent non-ionique à une concentration comprise entre 0,004% et 0,050%
∘ et/ou une solution de lyse comprenant une saponine à une concentration comprise entre 0,03% et 4%

• une endonucléase permettant de digérer les acides nucléiques libérés par l'action de la composition lysante
• une endopeptidase agissant à un pH compris entre 7 et 8,

- Filtrer les microorganismes viables potentiellement présents à travers un filtre dont le diamètre des pores est compris entre 0,30 et 0,50 µm.

La présente invention concerne également la méthode associée de détection en cytométrie en phase solide.

## Description

La présente invention concerne le domaine technique du contrôle microbiologique dans l'industrie pharmaceutique. En particulier l'invention a pour objet la préparation d'un échantillon de produits cellulaires pour une détection des microorganismes viables notamment par cytométrie en phase solide.

Les Médicaments de Thérapie Innovante (MTI) ou Advanced Therapy Medicinal Product (ATMP) représentent une classe de produits pharmaceutiques en pleine expansion. Aujourd'hui encore limités à quelques applications ciblées (hémopathies, maladies rares), mais ayant d'ores et déjà démontré leur efficacité, ils sont voués à prendre une place de plus en plus importante dans notre système de soins (plus d'un millier d'essais cliniques étaient en cours en 2020 dont près d'une centaine en phase III).

Ces médicaments reposent sur l'utilisation de produits cellulaires (autologues, allogéniques ou xénogéniques) qui ont été soumis à des manipulations substantielles ex vivo, modifiant leurs caractéristiques biologiques, leurs fonctions physiologiques ou leurs propriétés structurelles, avant de les administrer à l'homme.

La production et l'utilisation à grande échelle de ces médicaments se heurtent néanmoins à un grand nombre de difficultés, en particulier :
- Des coûts de production beaucoup plus élevés que pour les médicaments chimiques (nouveaux procédés de production complexes, faibles volumes produits...)
- Une stabilité du produit fini très courte, lorsqu'il est utilisé à l'état frais (de l'ordre de quelques heures) et donc la nécessité de pouvoir disposer de contrôles de qualité dans un délai très court
- L'impossibilité de les stériliser et donc un risque accru de contamination microbienne.

Les contrôles microbiologiques représentent une étape critique du cycle de production des MTI, en particulier les tests de stérilité conditionnant la libération. Ils sont décrits dans la Pharmacopée Européenne, section 2.6.27 « Microbiological examination of cell-based préparations ». Les contraintes de délai et de coûts associées à ces contrôles microbiologiques sont aujourd'hui l'un des principaux freins au développement de l'industrie des MTI.

Les tests de stérilité sont rendus obligatoires par la Pharmacopée européenne sur les produits finaux, conditionnant leur libération. La méthode traditionnelle, à savoir la culture cellulaire dans des bouillons et inspection visuelle, requiert un minimum de 14 jours pour assurer la non-contamination. Des méthodes alternatives, basées également sur la culture cellulaire, mais avec une lecture automatisée, ont été approuvées par la Pharmacopée, et nécessitent a minima 7 jours d'incubation, le plus souvent 10 jours, pour pouvoir rendre un résultat. La solution BACT/ALERT^{®} de bioMérieux, qui détecte la croissance de micro-organismes à travers la production de CO₂, constitue aujourd'hui la référence pour les contrôles de stérilité des MTI/ATMP.

Ces méthodes ont néanmoins des délais de réponse particulièrement longs ce qui peut impacter la vie des patients. Par ailleurs, elles requièrent souvent des prises d'échantillons peu compatibles avec les volumes produits dans le cadre des activités de fabrication de MTI, a fortiori en situation autologue.

Dans le but de réduire les délais mais également d'améliorer les capacités de détection, d'autres technologies sont actuellement à l'étude pour réaliser les tests de stérilité sur les MTI :
- Détermination de l'ATP (Adénosine triphosphate)
- Tests de biologie moléculaire.

Une autre technologie qui n'a pas été explorée pour les tests de stérilité sur les MTI est la cytométrie en phase solide. Un cytomètre en phase solide permet de quantifier très rapidement dans un échantillon les microorganismes tels que les bactéries, levures ou moisissures. L'échantillon est filtré sur une membrane afin que tout microorganisme présent dans l'échantillon soit récupéré sur la membrane. Les microorganismes vivants sont ensuite marqués spécifiquement par une molécule qui pénètre les microorganismes viables puis est clivée par les estérases ce qui libère un fluorochrome, la fluorescéine dans le microorganisme, émettant à une longueur d'onde spécifique. Un rayon laser balaye toute la surface de la membrane pour exciter le fluorochrome ce qui permet de détecter et compter les microorganismes viables éventuellement présents dans l'échantillon.

L'utilisation de la cytométrie en phase solide, appliquée aux tests de stérilité sur les MTI, suscite de nombreux défis. Les principales limites à cette technologie réside dans la forte concentration cellulaire égale ou supérieure à 10⁵ cellules/ml et dans la nature des cellules à lyser notamment les cellules immunitaires et leurs cellules progénitrices.

Ce problème de forte concentration cellulaire est rencontré dans la détection de bactéries dans les produits sanguins c'est à dire en général le sang total. Ainsi, le document WO03025207 décrit un procédé de concentration de bactéries à partir de produits sanguins qui consiste à réduire la concentration des populations des cellules sanguines au moyen d'une agrégation sélective de celles-ci. Une étape de filtration permet de recueillir dans le filtrat les germes pathogènes non agrégés et de retenir sur le filtre les agrégats de cellules sanguines puis éventuellement une lyse sélective des cellules résiduelles a lieu avant un nouveau passage sur filtre. L'étape de réduction par agrégation de la concentration des populations des cellules sanguines permet une réduction de l'ordre de 4 log (depuis 10⁹ à 10⁵ cellules/ml) en ce qui concerne la concentrations des plaquettes, et de l'ordre de 5 log (depuis 10¹⁰ à 10⁵ cellules/ml) en ce qui concerne la concentration des globules rouges. Bien que les microorganismes soient plus petits que les cellules sanguines agrégées, il existe un risque qu'ils soient retenus générant ainsi une perte des microorganismes et des faux négatifs par la méthode de détection.

Le problème de la forte concentration cellulaire est également rencontré dans les échantillons sanguins pour d'autres modes de détection telle qu'en biologie moléculaire. Les brevets EP2718713 et EP2510123 décrivent la lyse sélective de cellules sanguines dans un échantillon susceptible de contenir des microorganismes en utilisant des tampons de lyse maitrisés pour récupérer les microorganismes intacts morts ou vivants. L'ADN des microorganismes est ensuite extrait pour une détection par PCR. La viabilité des microorganismes n'est pas, pour une détection en biologie moléculaire, un critère essentiel. Ainsi, l'inconvénient de ces méthodes est que les tampons de lyse n'assurent pas la préservation de la viabilité des microorganismes nécessaire à une détection par cytométrie en phase solide avec une bonne sensibilité.

Zelenin (Biotechnol Lett 2015 37 : 825-830) décrit un protocole d'isolement de bactéries viables dans un échantillon sanguin utilisant de la saponine à une concentration entre 0,5% et 2,5% pour détruire les érythrocytes suivis d'un choc osmotique pour lyser les leucocytes. L'inconvénient de cette méthode est qu'elle n'est pas applicable à un échantillon à forte concentration en cellules immunitaires telles que les leucocytes beaucoup plus difficiles à lyser que les érythrocytes. Cette méthode d'isolement est suivie par une détection en biologie moléculaire et par des tests de sensibilité aux antibiotiques.

Le document EP3063290 décrit une méthode pour isoler les microorganismes d'un échantillon à forte densité cellulaire comprenant l'utilisation d'un tampon de lyse contenant 0,05 à 0,5% de SDS et de la Benzonase^{®} nuclease. Les échantillons contiennent 10⁶ cellules à 5×10⁸ cellules dans un échantillon d'au moins 5 ml. Les microorganismes sont ensuite lysés et détectés par biologie moléculaire. Cette méthode présente l'inconvénient de ne pas préserver la viabilité des microorganismes par l'utilisation d'un détergent anionique tel que le SDS.

D'autre part, le document EP2601304 décrit une préparation d'un échantillon sanguin pour une identification par spectrométrie de masse. Cela nécessite la lyse des particules humaines par de la saponine et une centrifugation ou une microfiltration pour ne récupérer que les microorganismes vivants. Ces microorganismes sont ensuite mis en culture, séparé du milieu de culture puis identifié par spectrométrie de masse. Après l'étape de centrifugation, une solution de saponine à 1% est ajouté au précipité qui contient les potentiels microorganismes mais aussi 5 × 10⁹ érythrocytes 7000 leucocytes et 50 000 plaquettes par millilitre de sang. Ce mélange est ensuite à nouveau centrifugé pour récupérer le précipité. Ce procédé n'est pas adapté à un échantillon à forte concentration en cellules immunitaires telles que les leucocytes, beaucoup plus difficile à lyser que les érythrocytes.

Une autre difficulté pour une application dans les MTI réside dans la filtrabilité des matrices cellulaires. Une solution consiste alors en un procédé de préparation pour rendre l'échantillon filtrable. Néanmoins ce procédé peut altérer de façon irréversible la charge microbienne soit en éliminant complètement des microbes, soit en les dégradant de telle sorte que le marquage de viabilité ne les détecte plus. Ainsi, le document WO2019051272 décrit la préparation d'un échantillon visqueux non filtrable pour une détection par cytométrie en phase solide. L'utilisation d'un solvant à base d'isopropyl myristate ne résout pas les problèmes rencontrés avec un échantillon à forte concentration cellulaire.

De même le document Journal of Microbiological Methods 64 (2006) 420-423, décrit la détection par cytométrie *d'aspergillus fumigatus* présent dans les sécrétions respiratoires, échantillon difficilement filtrable. Le prétraitement proposé comprend l'addition de rhDNAsel, de trypsine, de tampon HEPES, et de DTT/Triton X-100. L'utilisation de ce prétraitement adapté à un échantillon particulièrement visqueux ne résout pas les problèmes rencontrés avec un échantillon à forte concentration cellulaire.

Il existe donc un besoin de mettre au point une méthode de détection de microorganismes viables à partir d'échantillons comprenant une forte concentration de cellules immunitaires ou leurs cellules progénitrices, difficilement filtrable.

### RESUME DE L'INVENTION

Un objectif de la présente invention est de proposer un isolement de microorganismes viables potentiellement présents à très faible quantité dans un échantillon tout en éliminant les cellules immunitaires ou leurs cellules progénitrices présentes en très forte quantité.

Ainsi, un premier objet de l'invention concerne un procédé d'isolement de microorganismes viables potentiellement présents dans un échantillon comprenant 10⁶ à 10⁸ cellules immunitaires ou cellules progénitrices de ces dernières, comprenant les étapes suivantes :
- Mettre en contact l'échantillon avec
   - une composition lysante comprenant
      ∘ un tampon de lyse comprenant un détergent non-ionique à une concentration comprise entre 0,004% et 0,050%
      ∘ et/ou une solution de lyse comprenant une saponine à une concentration comprise entre 0,03% et 4%
   - une endonucléase permettant de digérer les acides nucléiques libérés par l'action de la composition lysante
   - une endopeptidase agissant à un pH compris entre 7 et 8,
- Filtrer les microorganismes viables potentiellement présents à travers un filtre dont le diamètre des pores est compris entre 0,30 et 0,50 µm.

La présente invention consiste donc à lyser de façon efficace les cellules immunitaires ou leurs cellules progénitrices présentes en très forte concentration tout en gardant les microorganismes viables et ainsi permettre leur détection par des méthodes qui nécessitent de garder viables les microorganismes. Préférentiellement, les cellules immunitaires sont des CAR-T.

Un avantage de la présente invention est qu'elle permet une préparation de l'échantillon efficace et rapide. Le temps nécessaire de mise en contact de l'échantillon avec la composition lysante, l'endonucléase et l'endopeptidase puis la fitration est de moins de 30 mn, préférentiellement moins de 20mn. Le procédé selon l'invention permet le maintien de la viabilité des microorganismes pour une détection notamment par cytométrie en phase solide qui est une détection particulièrement rapide. L'efficacité de la lyse selon l'invention est notamment due à la synergie entre la composition de lyse, l'endopeptidase et l'endonucléase. En effet, l'étape de lyse par la composition lysante induit la perte de viabilité des cellules immunitaires ou leurs cellules progénitrices. Elle agit en déstructurant et perméabilisant les membranes plasmiques des cellules, en formant des complexes avec les lipides des membranes. Ce mécanisme permet ensuite à l'endopeptidase d'hydrolyser les protéines membranaires et de faire éclater les cellules. L'action de l'endopeptidase se poursuit également afin de dégrader les membranes nucléaires et libérer le matériel intra-cellulaire. L'endonucléase, en dégradant les acides nucléiques, améliore également la lyse.

Préférentiellement, l'endopeptidase est une trypsine.

Préférentiellement, le détergent non-ionique est un détergent polyoxyéthylène tel que le BRIJ.

En présence d'une forte concentration cellulaire, l'échantillon est difficilement filtrable et l'efficacité de la lyse est alors un élément clé. Les petits débris obtenus par cette lyse permettent d'éviter que le filtre ne se colmate ce qui empêcherait la détection des microorganismes. Dans le cas d'une détection par cytométrie en phase solide, une lyse efficace évite d'avoir un bruit de fond de fluorescence important qui masquerait la présence de microorganismes.

Un autre objectif de la présente invention et de permettre une détection rapide par cytométrie en phase solide. La détection se fait entre 4h et 6h. Ainsi, un autre objet de l'invention concerne un procédé pour la détection de microorganismes potentiellement présents dans un échantillon comprenant 10⁶ à 10⁸ cellules immunitaires ou cellules progénitrices de ces dernières, comprenant les étapes suivantes :
- Effectuer le procédé d'isolement selon l'invention
- Effectuer un marquage des microorganismes viables retenus sur la membrane poreuse avec un marqueur de viabilité
- Effectuer un balayage par un faisceau laser ou par caméra de la membrane poreuse
- Déterminer la présence des microorganismes viables capturés par la membrane

Les cellules immunitaires ou leurs cellules progénitrices étant marquées comme les microorganismes par un marqueur de viabilité, la lyse complète des cellules immunitaires permet d'éviter le marquage de ces cellules et donc de réduire les faux positifs. De plus, la lyse permettant d'obtenir des débris cellulaires très petits, cela empêche l'adhésion des microorganismes sur ces débris et évite ainsi que les microorganismes ne soient masqués. Cette méthode réduit donc les faux négatifs.

Un autre objet de l'invention concerne un kit de lyse comprenant :
- un tampon de lyse comprenant un détergent non-ionique à une concentration comprise entre 0,004% et 0,050%
- une solution comprenant une saponine à une concentration comprise entre 0,03% et 4%
- une endonucléase
- une endopeptidase agissant à un pH compris entre 7 et 8.

Le kit de lyse selon l'invention permet une lyse efficace des cellules immunitaires ou leurs cellules progénitrices tout en étant non toxique pour les micro-organismes.

### DESCRIPTION DETAILLEE DE L'INVENTION

La présente invention concerne un procédé d'isolement de microorganismes viables potentiellement présents dans un échantillon comprenant une forte quantité de cellules immunitaires ou leurs cellules progénitrices, et la détection desdits microorganismes par cytométrie en phase solide.

Un premier objet de l'invention concerne un procédé d'isolement de microorganismes viables potentiellement présents dans un échantillon comprenant 10⁶ à 10⁸ cellules immunitaires ou cellules progénitrices de ces dernières, comprenant les étapes suivantes :
- Mettre en contact l'échantillon avec
   ∘ une composition lysante comprenant
      - un tampon de lyse comprenant un détergent non-ionique à une concentration comprise entre 0,004% et 0,050%
      - et/ou une solution de lyse comprenant une saponine à une concentration comprise entre 0,03% et 4%
   ∘ une endonucléase permettant de digérer les acides nucléiques libérés par l'action de la composition lysante
   ∘ une endopeptidase agissant à un pH compris entre 7 et 8.
- Filtrer les microorganismes viables potentiellement présents à travers un filtre dont le diamètre des pores est compris entre 0,30 et 0,50 µm.

L'échantillon selon la présente invention est un échantillon clinique comprenant 10⁶ cellules à 10⁸ cellules immunitaires ou leurs cellules progénitrices. Dans un mode de réalisation préféré, la quantité de cellules dans l'échantillon est de 10⁶ à 5×10⁷ cellules immunitaires ou leurs cellules progénitrices et encore plus préférentiellement 5×10⁶ et 10⁷ cellules. La quantité d'échantillon utilisée varie en fonction de la source d'échantillon. Dans un mode de réalisation préféré, le volume d'échantillon est de 100 µl à 2 ml encore plus préférentiellement de 500 µl à 1,5 ml.

Les cellules immunitaires ou les cellules progénitrices font partie des médicaments de thérapie innovante ou des produits de thérapie cellulaire. Les cellules immunitaires peuvent être choisies parmi les cellules NK, les monocytes, les lymphocytes B, les lymphocytes T, naturels ou génétiquement modifiés, tels que les lymphocytes T régulateurs, les lymphocytes T infiltrant les tumeurs, les lymphocytes T cytotoxiques, les lymphocytes T auxiliaires (ou helper) et les lymphocytes T ayant un récepteur antigénique chimérique (CAR).

Les cellules NK (ou lymphocytes NK) sont des cellules de l'immunité innée. Ce sont des lymphocytes non T (CD3-), non B (CD19-), caractérisés chez l'homme par les marqueurs CD56, CD16 et NK.

Les monocytes sont des leucocytes qui évoluent en macrophages, cellules dendritiques ou ostéoclastes.

Les lymphocytes B sont les cellules immunitaires responsables de la production d'anticorps.

Les lymphocytes T régulateurs sont une sous-population de lymphocytes T CD4+, qui inhibent la prolifération d'autres lymphocytes T effecteurs.

Les lymphocytes T cytotoxiques sont une sous-population de lymphocytes T CD8+, qui détruisent les cellules infectées.

Les lymphocytes T auxiliaires (helper) sont une sous-population de lymphocytes T CD4+, intermédiaires de la réponse immunitaire.

Enfin, les lymphocytes T ayant un récepteur antigénique chimérique (CAR), appelés également cellules CAR-T, correspondent à une technologie particulière d'ingénierie cellulaire. Ce sont des lymphocytes T qui expriment un récepteur antigénique chimérique. Les cellules CAR-T sont capables de tuer les cellules cancéreuses, par reconnaissance et liaison à l'antigène tumoral présent sur lesdites cellules cancéreuses. De préférence, l'échantillon selon l'invention comprend des CAR-T.

Les cellules progénitrices des cellules immunitaires peuvent être des cellules souches mésenchymateuses.

L'échantillon de cellules peut provenir du patient à traiter, le patient et le donneur sont alors la même personne, par biopsie ou prélèvement sanguin. Dans ce cas, la composition obtenue et conservée sera administrée à ce même patient : c'est un produit autologue.

Alternativement, l'échantillon de cellules peut provenir d'une autre source, comme un autre individu ou par ingénierie cellulaire, notamment par biopsie ou prélèvement sanguin. Dans ce cas, la composition obtenue et conservée sera administrée à un patient à traiter autre que le donneur : c'est un produit allogénique.

L'échantillon peut être conditionné frais dans un milieu liquide avec une conservation limitée à quelques heures ou bien sous forme d'une solution congelée. L'échantillon peut comprendre des cryoconservateurs. De préférence, la méthode selon l'invention, met en oeuvre un échantillon frais. L'échantillon peut être conservé avec de la BSA (bovine sérum albumine).

L'échantillon comprend ou est susceptible de contenir un microorganisme. Les microorganismes pouvant être isolés et/ou détectés selon la présente invention peuvent être des bactéries, des levures, des champignons. Les microorganismes peuvent être aérobies ou anaérobies. Les microorganismes détectés peuvent être par exemple *Corynebacterium tuberculosteariticum, Moraxella catarrhalis, Moraxella osloensis, Bacteroïdes fragilis Pichia carsonii Bretanomyces bruxellensis Paracoccus yeei, Sacharomyces cerevisiae, Streptococcus pyogenes, Pénicillium expensum, Sphingomonas paucimobilis, Kocuria rhizophila, Staphylococcus epidermidis, Aspergillus brasiliensis, Candida albicans, Bacillus subtilis, Staphylococcus aureus, Escherichia coli, Pseudomonas aeruginosa, Cutibacterium acnes, Clostridium sporogenes.*

Afin d'isoler les microorganismes, la présente invention propose une méthode d'isolement des microorganismes directement à partir de l'échantillon. L'échantillon de cellules immunitaires ou leurs cellules progénitrices est directement mis en contact avec une composition de lyse. L'échantillon de cellules immunitaires ou leurs cellules progénitrices ne subit pas préalablement de centrifugation, filtration ou encore d'agrégation sélective pour réduire la concentration desdites cellules.

En effet, ces étapes préalables peuvent engendrer une perte des microorganismes et générer des faux négatifs. La centrifugation à vitesse élevée, par exemple, peut favoriser un phénomène d'adhésion entre les cellules immunitaires et certaines bactéries, empêchant leur séparation.

Selon la présente invention, il est donc nécessaire d'effectuer une lyse sélective détruisant les cellules immunitaires ou leurs cellules progénitrices afin de récupérer les microorganismes viables. Ainsi, l'ajout d'un détergent chimique ou surfactant est couramment pratiqué afin de solubiliser les protéines membranaires des cellules mammifères. Les détergents sont des composés amphiphiles capables de former des micelles en solution aqueuse, à l'intérieur desquelles se trouvent les têtes hydrophobes et à l'extérieur desquelles se trouvent les têtes hydrophiles. Ces micelles désorganisent les membranes phospholipidiques. Les détergents peuvent être ioniques comme le Sodium dodecyl sulphate (SDS), non-ioniques et plus doux comme le Triton X ou zwitterioniques.

La paroi des bactéries est solide, elle consiste essentiellement en peptidoglycane. Dans le cas des gram +, il y a également de l'acide teichoïque. Ce réseau de liaisons covalentes résiste à l'effet de dissolution des surfactants pendant plusieurs minutes. Néanmoins en fonction de la quantité et de la nature du surfactant, ces derniers peuvent pénétrer à l'intérieur des microorganismes et détruire la structure des protéines impactant leur capacité à se reproduire et donc leur viabilité.

Toute la difficulté de la lyse sélective réside sur le choix d'une composition de lyse à la fois efficace pour lyser les cellules immunitaires ou leurs cellules progénitrices et non toxique pour les micro-organismes. Dans la présente invention, il est essentiel que les microorganismes soient viables. Ainsi, il est nécessaire d'utiliser un surfactant faible non toxique.

D'autre part, l'efficacité de la composition de lyse dépend de la quantité de cellules présente dans l'échantillon. Or l'échantillon de la présente invention est composé d'une très forte concentration cellulaire. Elle dépend également de la nature de l'échantillon. Une composition de lyse efficace pour des érythrocytes ne l'est pas forcément pour des cellules immunitaires telles que les leucocytes. En effet, le sang est composé majoritairement d'érythrocytes et d'environ 4000 à 7000 leucocytes par ml de sang. Or les érythrocytes sont de petites cellules qui se lysent plus facilement que les leucocytes.

Ainsi, selon la présente invention, l'échantillon est mis en contact avec
- une composition lysante comprenant
   ∘ un tampon de lyse comprenant un détergent non-ionique à une concentration comprise entre 0,004% et 0,050%
   ∘ et/ou une solution de lyse comprenant une saponine à une concentration comprise entre 0,03% et 4%
- une endonucléase permettant de digérer les acides nucléiques libérés par l'action de la composition lysante
- une endopeptidase agissant à un pH compris entre 7 et 8,

La composition de lyse est utilisée pour rompre les membranes cellulaires en jouant sur leurs propriétés physico-chimiques.

Préférentiellement, la composition de lyse comprend de la saponine. On citera la Saponine Quillaja. Dans le cas des leucocytes, la membrane est plus robuste que celle des érythrocytes. De plus les cellules immunitaires ou cellules progénitrices présentent également un noyau et des organelles intracellulaires qui les rendent plus résistantes à la lyse. Préférentiellement, la concentration finale en saponine est comprise entre 0,03% et 2%, encore plus préférentiellement entre 0,05% et 1%, encore plus préférentiellement entre 0,05% et 0,8 %, encore plus préférentiellement entre 0,05% et 0,2 %.

Préférentiellement, la composition de lyse contient un détergent non ionique. De préférence le détergent non ionique est un détergent polyoxyéthylène. De préférence, ledit détergent est le BRIJ. On peut citer le BrijO10. Préférentiellement, la concentration finale en détergent non ionique est comprise entre 0,004% à 0,050%, plus préférentiellement entre 0,004% à 0,030%.

Lorsqu'il n'y a pas de saponine, la concentration finale du détergent non ionique est entre 0,025% et 0,05%.

Lorsqu'il n'y a pas de détergent, la concentration finale de la saponine est entre 0,10% et 4% saponine.

Les cellules immunitaires ou les cellules progénitrices de ces dernières, présentes à très forte concentration, sont lysées partiellement avec de la saponine à faible concentration et/ou un détergent non ionique à faible concentration, sans pour autant détruire les microorganismes présents en très faible quantité. Avantageusement, afin de ne pas dégrader les microorganismes, la composition de lyse ne contient pas de détergents ioniques tels que le SDS.

La préparation de la composition de lyse et notamment du tampon de lyse comprenant un détergent non ionique est connue de l'homme du métier. Les ingrédients sont dissous et mixés.

L'échantillon est également mis en contact avec une endopeptidase agissant efficacement à un pH compris entre 7 et 8,5, préférentiellement, elle agit à un pH entre 7 et 8. L'endonucléase permet d'obtenir une lyse complète des cellules immunitaires. En effet, le détergent non ionique polyoxyéthylène tel que le BRIJ et/ou la saponine déstructure et perméabilise les membranes plasmiques des cellules immunitaires et cellules progénitrices de ces dernières, en formant des complexes avec les lipides des membranes. Ce mécanisme permet ensuite à l'endopeptidase d'hydrolyser les protéines membranaires et de faire éclater les cellules.

L'endopeptidase utilisée selon l'invention n'altère pas la survie des microorganismes. On citera comme endopeptidase par exemple la trypsine ou encore un mélange d'endopeptidase tel que la composition accutase^{®} de biowest. Préférentiellement, l'endopeptidase est une trypsine. Elle a une activité optimale à pH 8. La trypsine peut être sous forme liquide ou sous forme de poudre que l'on remet en solution. Préférentiellement la concentration finale en trypsine est en excès. Elle peut être, par exemple supérieure à 0,1%.

Selon la présente invention, la détection peut être réalisée grâce à un marqueur de viabilité. Or, les cellules immunitaires ou leurs cellules progénitrices pouvant être marquées comme les microorganismes par un marqueur de viabilité, la lyse complète des cellules immunitaires permet de réduire les faux positifs. De plus, la lyse permettant d'obtenir des débris cellulaires très petits, cela empêche l'adhésion des microorganismes sur ces débris et évite ainsi que les microorganismes ne soient masqués. Cette méthode réduit donc les faux négatifs.

Dans un mode de réalisation, l'échantillon est mis en contact avec une endopeptidase avant la mise en contact avec la composition de lyse. Ce mode de réalisation est particulièrement avantageux lorsque l'échantillon comprend de la BSA.

Le pH est également à prendre en compte pour optimiser l'efficacité des agents de lyse. Typiquement, les agents de lyse ont un pH compris entre 5 et 9. Préférentiellement, les agents de lyse agissent à un pH entre 7 et 8,5, plus préférentiellement entre 7 et 8.

Ainsi, à pH basique entre 7 et 8, la saponine et la trypsine ont une très bonne efficacité. De plus à ce pH, la viabilité des microorganismes cibles est maintenue, ce qui n'est pas le cas pour certains microorganismes cibles au-dessus d'un pH de 8.5.

Après l'ajout de l'endopeptidase, une étape de rinçage avec un fluide approprié est effectué afin de stopper les réactions enzymatiques et chimiques. Préférentiellement, le fluid D est utilisé.

La composition de lyse et l'endopeptidase sont ajoutées à l'échantillon pendant une durée appropriée et en fonction de leur quantité pour lyser les cellules immunitaires ou leurs cellules progénitrices tout en préservant les microorganismes potentiellement présents, vivants.

L'échantillon est également mis en contact avec une ou des enzymes permettant de lyser les acides nucléiques. Ces enzymes sont connues et classiquement utilisées par l'homme du métier. L'endonucléase dégrade de façon non spécifique toutes les formes d'ADN et d'ARN. On peut citer par exemple la Benzonase^{®} Nuclease disponible commercialement par la société Merck Millipore. De préférence, la concentration finale d'endonucléase est en excès. La concentration finale peut être comprise entre 50 Ul/ml et 100 Ul/ml.

L'endonucléase peut être séparée de la composition lysante ou faire partie de la composition lysante. Elle peut être ajoutée avant, parallèlement ou après la composition lysante et l'endoptidase. L'endopeptidase peut éventuellement être inactivée avant l'ajout de l'endonucléase.

L'endonucléase en dégradant les acides nucléiques évite au filtre d'être colmaté. La filtration est donc améliorée évitant des faux positifs.

L'endonucléase peut être sous forme séchée.

Après l'étape de mise en contact de l'échantillon avec la composition de lyse et l'endopeptidase, l'ensemble est homogénéisé ou mixé c'est-à-dire par vortex ou agitation. De plus une étape d'incubation sous agitation peut avoir lieu pendant quelques minutes.

Les microorganismes viables potentiellement présents sont ensuite séparés de l'échantillon par filtration. La séparation est effectuée sur une membrane poreuse ayant des pores inférieurs à la taille des bactéries. Préférentiellement, la membrane poreuse a des pores compris entre 0,30 et 0,50 µm, préférentiellement entre 0,32 et 0,45 µm. Ainsi les bactéries dont le diamètre est inférieur à celui des levures et moisissures sont retenues par le filtre. Du fait du diamètre très faible de la membrane, la lyse de l'échantillon doit être très efficace pour ne pas colmater le filtre et entrainer un fond de fluorescence très important qui peut masquer la fluorescence spécifique des micro-organismes. Cette méthode réduit donc les faux négatifs.

L'étape d'isolement des microorganismes est une étape rapide de moins de 30 mn, préférentiellement moins de 20mn.

Après l'isolement des microorganismes, leur détection peut avoir lieu par tout type de méthode telle que la biologie moléculaire ou encore par cytometrie. La détection selon l'invention est effectuée par cytométrie en phase solide permettant une détection des microorganismes viables potentiellement présents.

Ainsi, un autre objet de l'invention concerne un procédé pour la détection de microorganismes potentiellement présents dans un échantillon comprenant 10⁶ à 10⁸ cellules immunitaires ou leurs cellules progénitrices, comprenant les étapes suivantes :
- effectuer le procédé d'isolement selon l'invention
- effectuer un marquage des microorganismes viables retenus sur la membrane poreuse avec un marqueur de viabilité
- effectuer un balayage par un faisceau laser ou par caméra de la membrane poreuse
- déterminer la présence des microorganismes viables capturés par la membrane

La détection selon la présente invention est particulièrement rapide et se fait entre 4h et 6h, la détection des microorganismes aérobies étant plus rapide que celle des microorganismes anaérobies qui nécessite des temps d'incubation plus longs. La cytométrie en phase solide est une technologie prometteuse pour les tests de stérilité sur les MTI. Elle permet une détection très sensible des microorganismes. Ainsi, la technologie SCANRDI^{®} développée par la demanderesse permet de détecter toute présence de microorganismes dans un échantillon filtrable avec un rendu de résultats très rapide. Le protocole selon l'invention permet ainsi un test de stérilité ultra rapide d'un échantillon de cellules immunitaires ou leurs cellules progénitrices en moins de 6h. Ce cytomètre en phase solide permet de quantifier très rapidement les microorganismes (bactéries, levures ou moisissures) dans un échantillon. L'échantillon est filtré sur une membrane afin que tout microorganisme présent dans l'échantillon soit récupéré sur la membrane. Les microorganismes vivants sont ensuite marqués spécifiquement par un marqueur de viabilité qui pénètre les microorganismes puis est clivé par les estérases ce qui libère un fluorochrome, la fluorescéine dans le microorganisme, émettant à une longueur d'onde spécifique. Les cellules immunitaires ou leurs cellules progénitrices contiennent également des estérases et peuvent absorber le substrat de la même façon que les microorganismes. Leur marquage à la fluorescéine peut alors entrainer des faux positifs. La lyse des cellules est donc indispensable pour distinguer les microorganismes présents dans l'échantillon des cellules immunitaires ou leurs cellules progénitrices. Après l'étape de marquage, un rayon laser balaye toute la surface de la membrane pour exciter le fluorochrome ce qui permet de détecter et compter les microorganismes viables éventuellement présents dans l'échantillon. Un microscope couplé au Scan permet de valider visuellement la présence de microorganismes. La présente invention permet d'atteindre une limite de détection de 1CFU par échantillon.

D'autre part, un défi pour l'utilisation de cette technologie pour une application aux tests de stérilité sur les médicaments de thérapies innovantes réside dans la présence d'une forte concentration de cellules égale ou supérieure à 10⁵ cellules/ml, de préférence entre 10⁶ à 5×10⁷ cellules/ml. En effet, cette matrice non filtrable entraine sur la membrane un fond de fluorescence très important qui peut masquer la fluorescence spécifique des micro-organismes dont la quantité est très faible. Il est donc nécessaire d'avoir une lyse très efficace pour obtenir des débris très petits. La membrane poreuse du cytomètre en phase solide permet de retenir les microorganismes tout en permettant au fluide et débris cellulaires de la traverser. La membrane correspond à une pluralité de pores ayant un diamètre moyen inférieur à 0,50 µm, préférentiellement entre 0,32 à 0, 45 µm.

Un autre objet de l'invention concerne un kit de lyse comprenant :
- un tampon de lyse comprenant un détergent non-ionique à une concentration comprise entre 0,004% et 0,050%
- une solution comprenant une saponine à une concentration comprise entre 0,03% et 4%
- une endonucléase
- une endopeptidase agissant à un pH compris entre 7 et 8

Dans une méthode alternative, la détection peut être réalisée par biologie moléculaire. Il s'agit alors d'un procédé pour la détection de microorganismes potentiellement présents dans un échantillon comprenant 10⁶ à 10⁸ cellules immunitaires ou leurs cellules progénitrices, comprenant les étapes suivantes :
- effectuer le procédé d'isolement selon l'invention
- incuber l'échantillon dans un milieu d'enrichissement permettant la croissance des microorganismes
- éliminer ou inactiver l'endonucléase
- lyser les microorganismes pour récupérer les acides nucléiques
- mettre en contact la solution d'acides nucléiques avec des amorces permettant d'amplifier au moins un gène ou un fragment de gène dudit microorganismes
- détecter ledit microorganisme

L'étape d'isolement des microorganismes viables permet d'éliminer les ADN résiduels.

L'inactivation de l'endonucléase peut se faire par dilution dans un grand volume. Elle peut avoir lieu avant ou après l'incubation dans le milieu d'enrichissement.

L'invention est illustrée avec les exemples non-limitatifs présentés ci-après.

### Exemples

### Exemple 1 : Préparation des agents lysants

Un tampon de lyse est préparé avec :
- Trizma: 6 g
- MgCl₂ :1,904g
- BrijO10 : 0,1296g
- H₂O: 900 ml
- Thiosulfate de sodium : ajout de 1 ml de la solution mère. ( 0,0499 g/L )

Le pH du tampon est ajusté à 8.

Dans ce tampon, 1,5g/l de saponine (Saponine Quillaja S4521-10G, Sigma) sont préparées.

4µl de benzonase (Benzonase E1014-25KU, Sigma) sont ajoutés à 7ml de la solution préalablement préparée.

2ml de trypsine, (Trypsine EDTA : ref 25200-072-500ml, Sigma) seront ajoutés pendant le protocole de lyse.

### Exemple 2 : Préparation de l'échantillon de cellules immunitaires ou leurs cellules progénitrices

1 ml de cellules dans du milieu RPMI 1640 (ref MS0A6Y100A biowest) est mis en contact avec 5 CFU de micro-organismes (100µl).

Les cellules immunitaires utilisées sont des cellules JURKAT ou des cellules CAR-T :
- cellules JURKAT (clone E6-1, ATCC TIB-152 ou ECACC 88042803), 1 ml, à 10⁷ cellules /ml,
- les cellules CAR-T (ProMab PM-CAR2003-2M), 0,5ml, à 2×10⁶ cellules/ml,
- les cellules CAR-T (ProMab PM-CAR2003-2M), 1 ml, à 4×10⁶ cellules/ml,
- les cellules T (cellules de patients Etablissement français du sang), 1 ml, à 2×10⁷ cellules/ml.

Les cellules progénitrices sont :
- les cellules souches mésenchymateuses du cordon ombilical (cellules de patients), 1 ml avec 1×10⁷ cellules,
- les lymphocytes , 1 ml avec 2×10⁷ cellules,
- les cellules souches mésenchymateuses de moelle osseuse, 1,5 ml avec 1,5×10⁶ cellules.

### Exemple 3 : Protocole de lyse

L'échantillon contaminé est mis en contact avec 1 ml de la composition de lyse et la benzonase.

Cette solution est mise à incuber à 37°C pendant 5 minutes avec une agitation de 1000 RPM dans un ThermoMixer C Ependorf SN (37°C). Après les 5 minutes d'incubation, 2 ml de trypsine sont ajoutés et ce mélange est de nouveau mis sous agitation à 1000 RPM et à 37°C pendant 5 minutes. La solution est ensuite diluée avec de l'ajout de quelques ml de fluiD (ref 42624 BioMérieux) pour stopper les réactions chimiques et enzymatiques.

### Exemple 4 : Détection de microorganismes dans l'échantillon lysé

Cette solution est ensuite filtrée sur membrane dont la taille des pores est comprise entre 0,32 et 0,38 µm sur le dispositif SCANRDI^{®} (membrane CB04 et ScanFilter 415701). Plusieurs lavages sont possibles avec du FluiD.

1ml de solution de contre-colorants nommés CSE/CSM ((CSE ref 205-R4070-01bioMérieux /CSM 205-R4109-01) dilution au 1/100 de CSE dans le CSM) est ajouté et laissé sur la membrane du SCANRDI^{®} pendant 10 secondes puis le CSE/CSM est ensuite passé à travers la membrane.

La membrane est ensuite récupérée et déposée sur le PAD d'activation sur lequel est déposé le milieu d'activation (CELL BURST ACTIVATE kit bioMérieux ref 424512 : CELL BURST ACTIVATE AER équivalent à un bouillon TSB pour les germes aérobies, CELL BURST ACTIVATE ANAER équivalent à un bouillon thioglycolate pour les germes anaérobies). Le PAD est placé dans un incubateur à 30°C pour 2h pour les germes aérobies et 2h30 pour les germes anaérobies sous ambiance anaérobie (GEN bag Anaer bimérieux 455534).

Les PAD sont ensuite changés et un nouveau PAD avec le marqueur (ChemChrome V6 ref 201-R1007-03 dilué au 1/100 dans du ChemSol B16 205-R2023-02) est incubé 45 minutes pour les germes aérobies et 1h30 pour les germes anaérobies sous ambiance anaérobie.

Le marqueur utilisé dans la technologie du SCANRDI^{®} est un marqueur de viabilité. Le SCANRDI^{®} ne détecte que les germes viables.

Au terme de l'incubation, les membranes sont récupérées et analysées au SCANRDI^{®}.

### Exemple 5 : Résultats obtenus avec le procédé de détection selon l'invention des microorganismes aérobies

La préparation de l'échantillon, le protocole de lyse (avec les cellules JURKAT) et la détection sont décrits aux l'exemples 1, 2, 3 et 4. Les microorganismes utilisés sont : *Staphylococcus aureus, Pseudomonas aeruginosa, bacillus subtilis, Streptococcus pyogenes, kocuria rhyzophila.* Chaque micro-organisme est testé 5 fois (5 réplicats) et en parallèle avec 5 boites de Pétri pour vérifier la justesse de l'inoculum (5CFU) sur boites.

| Microorganimses | Résultats SCANRDI^{®} obtenus en moyenne sur 5 replicats | Résultats boite de Pétri obtenus en moyenne sur 5 replicats |
|---|---|---|
| *Staphylococcus aureus* | 3 | 3,6 |
| *Pseudomonas aeruginosa* | 4 | 5,2 |
| *bacillus subtilis* | 4 | 6 |
| *Streptococcus pyogenes* | 2 | 2 |
| *kocuria rhyzophila* | 5,4 | 6,8 |

Les résultats montrent que les microorganismes inoculés avec les cellules pour subir la lyse, sont restés viables et ont été détectés au SCANRDI^{®}. Les résultats obtenus montrent que 5 CFU peuvent être détectés pour les microorganismes testés.

### Exemple 6 : Résultats obtenus avec le procédé de détection selon l'invention des microorganismes anaérobies

La préparation de l'échantillon, le protocole de lyse (avec les cellules JURKAT) et la détection sont décrits à l'exemple 1, 2, 3 et 4 spécifique pour les anaérobies. Les microorganismes utilisés sont : *bacteroides fragilis, clostridium sporogenes, cutibacterium acnes*

| Microorganismes | Résultats SCANRDI^{®} obtenus en moyenne sur 5 replicats | Résultats boite de Pétri obtenus en moyenne sur 5 replicats |
|---|---|---|
| *Bacteroides fragilis* | 6 | 7 |
| *Clostridium sporogenes* | 6 | 5,2 |
| *Cutibacterium acnes* | 3 | 4,6 |

Les résultats montrent que les microorganismes anaérobies qui ont été inoculés avec les cellules pour subir la lyse, sont restés viables et ont été détectés au SCANRDI^{®}. La présence du thioglycolate dans le protocole de détection a permis une très bonne récupération.

Les résultats obtenus montrent que 5 CFU peuvent être détectés pour les microorganismes testés.

### Exemple 7 : Résultats obtenus avec le procédé de détection selon l'invention des microorganismes anaérobies Candida albicans et Aspergillus brasiliensis

La préparation de l'échantillon, le protocole de lyse (avec les cellules JURKAT) et la détection sont décrits à l'exemple 1, 2, 3 et 4. Les microorganismes utilisés sont : *Candida albicans, Aspergillus brasiliensis.*

| Microorganismes | Résultats SCANRDI^{®} obtenus en moyenne sur 5 replicats | Résultats boite de Pétri obtenus en moyenne sur 5 replicats |
|---|---|---|
| *Candida albicans* | 3 | 4,6 |
| *Aspergillus brasiliensis* | 3,4 | 4 |

Les résultats montrent que les microorganismes anaérobies inoculés avec les cellules pour subir la lyse, sont restés viables et ont été détectés au SCANRDI^{®}. Les résultats obtenus montrent que 5 CFU peuvent être détectés pour les germes testés.

### Exemple 8 : Efficacité de la lyse de cellules JURKAT en fonction de la concentration en saponine, BRIJO10 et trypsine

| | | | | |
|---|---|---|---|---|
| Quantité de cellules JURKAT | 1.10⁷ | 1.3 10⁷ | 1.6 10⁷ | 1.9 10⁷ |
| Volume de tampon de lyse | 1ml | 1ml | 1ml | 1ml |
| Volume total de la réaction | 2 ml | 2,3 ml | 2,6 ml | 2,9 ml |
| % de saponine | 0,075% | 0,065% | 0,0576% | 0,0517% |
| % de BRIJ O10 | 0,007% | 0,006% | 0,0049% | 0,0044% |
| Vol trypsine à 0,25% | 2 ml : 0,125% | 2 ml : 0 ,115% | 2 ml :0,107% | 2 ml :0,10% |
| Lecture au SCAN RDI | Lyse des cellules | Lyse des cellules | Lyse des cellules | Lyse des cellules |

Les résultats montrent que les cellules immunitaires (cellules JURKAT) peuvent être lysées de façon efficace.

### Exemple 9 : Influence de la concentration en Saponine et BRIJ O10 sur la viabilité des microorganismes.

Selon l'exemple 1, exemple 2 et l'exemple 3, la toxicité du protocole a été testée avec différents microorganismes. L'échantillon est constitué de cellules JURKAT.

A la suite de l'exemple 3 la solution est filtrée sur un filtre NALGENE comportant une membrane dont le diamètre des pores est de 0,45 µm pour récupérer les microorganismes et les faire croitre dans des milieux sur boite de Pétri adaptés aux types de microorganismes.

| souche | Volume de Cellules | % de saponine | % en BRIJ | % récupération sur filtre versus moyenne inoculée |
|---|---|---|---|---|
| Staphylococcus aureus (BB) | 0,1ml | 0,1364% | 0,0127% | 126,26% |
| | 0,4ml | 0,1071% | 0,0100% | 126,26% |
| | 0,7ml | 0,0882% | 0,0082% | 142,42% |
| | 1 ml | 0,075% | 0,0070% | 155,55% |
| Pseudomonas aeruginosa (BB) | 0,1ml | 0,1364% | 0,0127% | 100,53% |
| | 0,4ml | 0,1071% | 0,0100% | 95,14% |
| | 0,7ml | 0,0882% | 0,0082% | 89,06% |
| | 1 ml | 0,075% | 0,0070% | 82,32% |
| Bacillus subtilis (BB) | 0,1ml | 0,1364% | 0,0127% | 80,74% |
| | 0,4ml | 0,1071% | 0,0100% | 80,74% |
| | 0,7ml | 0,0882% | 0,0082% | 83,33% |
| | 1 ml | 0,075% | 0,0070% | 87,85% |
| Aspergillus brasiliensis (BB) | 0,1ml | 0,1364% | 0,0127% | 99,25% |
| | 0,4ml | 0,1071% | 0,0100% | 90,82% |
| | 0,7ml | 0,0882% | 0,0082% | 94,56% |
| | 1 ml | 0,075% | 0,0070% | 87,07% |
| Streptococcus pyogenes (BB) | 0,1ml | 0,1364% | 0,0127% | 108,69% |
| | 0,4ml | 0,1071% | 0,0100% | 114,49% |
| | 0,7ml | 0,0882% | 0,0082% | 124,63% |
| | 1 ml | 0,075% | 0,0070% | 121,73% |
| Kocurria rhizophila (BB) | 0,1ml | 0,1364% | 0,0127% | 100 |
| | 0,4ml | 0,1071% | 0,0100% | 98,80% |
| | 0,7ml | 0,0882% | 0,0082% | 105,95% |
| | 1 ml | 0,1500% | 0,0140% | 98,80% |
| Clostridium sporogenes (BB550) | 0,1ml | 0,1364% | 0,0127% | 80,49% |
| | 0,4ml | 0,1071% | 0,0100% | 90,32% |
| | 0,7ml | 0,0882% | 0,0082% | 70,66% |
| | 1 ml | 0,075% | 0,0070% | 81,10% |
| Bacteroides fragilis (prec) | 0,1ml | 0,1364% | 0,0127% | 89,70% |
| | 0,4ml | 0,1071% | 0,0100% | 92,39% |
| | 0,7ml | 0,0882% | 0,0082% | 94,63% |
| | 1 ml | 0,075% | 0,0070% | 84,34% |
| Cutibacterium acnes (BB) | 0,1ml | 0,1364% | 0,0127% | 102,40% |
| | 0,4ml | 0,1071% | 0,0100% | 96,38% |
| | 0,7ml | 0,0882% | 0,0082% | 94,37% |
| | 1 ml | 0,075% | 0,0070% | 102,40% |
| Moraxella catharralis (prec) | 0,1ml | 0,1364% | 0,0127% | 89,34% |
| | 0,4ml | 0,1071% | 0,0100% | 72,16% |
| | 0,7ml | 0,0882% | 0,0082% | 81,32% |
| | 1 ml | 0,075% | 0,0070% | 74,45% |
| Sphingomonas paucimobilis (prec) | 0,1ml | 0,1364% | 0,0127% | 92,59% |
| | 0,4ml | 0,1071% | 0,0100% | 86,20% |
| | 0,7ml | 0,0882% | 0,0082% | 98,97% |
| | 1 ml | 0,075% | 0,0070% | 85,56% |
| Escherichia coli (BB) | 0,1ml | 0,1364% | 0,0127% | 103,99% |
| | 0,4ml | 0,1071% | 0,0100% | 99,17% |
| | 0,7ml | 0,0882% | 0,0082% | 99,17% |
| | 1 ml | 0,075% | 0,0070% | 97,79% |
| Staphylococcus epidermidis (BB 30) | 0,1ml | 0,1364% | 0,0127% | 105,12% |
| | 0,4ml | 0,1071% | 0,0100% | 102,56% |
| | 0,7ml | 0,0882% | 0,0082% | 101,28% |
| | 1 ml | 0,075% | 0,0070% | 112,82% |
| Streptococcus pneumoniae (prec) | 0,1ml | 0,1364% | 0,0127% | 101,59% |
| | 0,4ml | 0,1071% | 0,0100% | 94,28% |
| | 0,7ml | 0,0882% | 0,0082% | 85,32% |
| | 1 ml | 0,075% | 0,0070% | 94,28% |
| Pénicillium expansum | 0,1ml | 0,1364% | 0,0127% | 65,36% |
| | 0,4ml | 0,1071% | 0,0100% | 61,47% |
| | 0,7ml | 0,0882% | 0,0082% | 74,45% |
| | 1 ml | 0,075% | 0,0070% | 63,20% |

Les résultats obtenus dans le tableau ci-dessus montre qu'à des concentrations de Saponine de 0,075 à 0,1364% et des concentrations de BRIJ comprises entre 0,007à 0 ,0127% il n'y a pas de toxicité pour les germes testés tout en permettant la lyse des lymphocytes T.

### Exemple 10 : Lyse de cellules Jurkat (Lymphocytes T) par la composition de lyse et/ou l'endopetidase

### 1) Echantillons de cellules Jurkat testés

Culture de cellules Jurkat bioMérieux, récolte du 15/09/2022, conservées dans 5% de DMSO + 10% BSA décongelées le 16/09/2022.

Les cellules sont diluées au 1/10ème soit à 10⁷ cellules/ml (1 ml de cellules dans 9 ml de RPMI).

### 2) Lyse cellulaire

La lyse est réalisée en suivant les formulations de l'exemple 1. On ajoute 1 ml de cellules et 1 ml de composition de lyse et d'endonucléase pendant 5 min à 37 °C sous agitation à 1000 rpm (triplicate). Après l'étape de lyse, 200 µl des lysats sont prélevés dans un premier tube afin de réaliser la mesure sur le système de cytométrie Gallios (beckman Coulter, Gallios 10 couleurs).

Dans le tube restant, on ajoute 2 ml de solution d'endopeptidase à 0.25%. Le tube est incubé 5 minutes à 37°C, puis, comme précédemment, 200 µl des lysats sont prélevés afin de réaliser la mesure sur le système Gallios.

### 3) Cytométrie en flux

Le cytomètre en flux Gallios est utilisé pour observer l'effet de la lyse sur les cellules aux 2 étapes.

Le tableau ci-dessous montrent le profil cytométrique de cellules n'ayant pas subies de lyse, de cellules ayant subies une lyse par la composition lysante avec l'endonucléase, et de cellules ayant subies une lyse par la composition lysante, l'endonucléase et l'endoptidase.

| | Cellules non traitées (%) | Cellules ayant subies une lyse par la composition de lyse et endonucléase (%) | Cellules ayant subies une lyse par la composition de lyse, l'endonucléase et l'endopeptidase (%) |
|---|---|---|---|
| % de cellules JURKAT vivantes | 52,58 | 3,39 | 0,10 |
| % de cellules JURKAT mortes | 13,89 | 52,40 | 2,20 |
| % de cellules JURKAT sous forme de débris cellulaires | 31,49 | 39,52 | 96,72 |

On observe 3 groupes de cellules :
- un groupe de cellules Jurkat vivantes
- un groupe de cellules Jurkat mortes
- un groupe de débris cellulaires

L'étape de lyse par la composition lysante et l'endonucléase (colonne 3) induit la perte de viabilité des cellules Jurkat. En effet, un déplacement de la quasi-totalité des cellules de l'état viable vers la fenêtre des cellules mortes est observé au cytomètre de flux. On observe une réduction de la taille des cellules correspondant à l'action de la composition de lyse sur les membranes cellulaires. La saponine et le BRIJ présents dans la composition de lyse agissent en déstructurant et perméabilisant les membranes plasmiques des cellules, en formant des complexes avec les lipides des membranes.

Ce mécanisme permet ensuite à l'endopeptidase d'hydrolyser les protéines membranaires et de faire éclater les cellules (colonne 4). Ainsi, on observe au cytomètre de flux un déplacement de la quasi-totalité des cellules mortes vers la fenêtre des débris cellulaires. L'action de la trypsine se poursuit également afin de dégrader les membranes nucléaires et libérer le matériel intra-cellulaire.

En conclusion, la composition de lyse et l'endopeptidase agissent en synergie permettant une meilleure lyse des cellules immunitaires.

## Revendications

**1.** Procédé d'isolement de microorganismes viables potentiellement présents dans un échantillon comprenant 10⁶ à 10⁸ cellules immunitaires ou cellules progénitrices de ces dernières, comprenant les étapes suivantes :
- Mettre en contact l'échantillon avec
• une composition lysante comprenant
∘ un tampon de lyse comprenant un détergent non-ionique à une concentration comprise entre 0,004% et 0,050%
∘ et/ou une solution de lyse comprenant une saponine à une concentration comprise entre 0,03% et 4%
• une endonucléase permettant de digérer les acides nucléiques libérés par l'action de la composition lysante
• une endopeptidase agissant à un pH compris entre 7 et 8,
- Filtrer les microorganismes viables potentiellement présents à travers un filtre dont le diamètre des pores est compris entre 0,30 et 0,50 µm.

**2.** Procédé selon l'une quelconque des revendications précédentes **caractérisé en ce que** l'échantillon comprend 10⁶ à 5×10⁷ cellules immunitaires ou cellules progénitrices de ces dernières, préférentiellement entre 5×10⁶ et 10⁷ cellules.

**3.** Procédé selon l'une quelconque des revendications précédentes **caractérisé en ce que** les cellules immunitaires sont des CAR-T.

**4.** Procédé selon l'une quelconque des revendications précédentes **caractérisé en ce que** l'endopeptidase est une trypsine.

**5.** Procédé selon l'une quelconque des revendications précédentes **caractérisé en ce que** le détergent non-ionique est un détergent polyoxyéthylène.

**6.** Procédé selon l'une quelconque des revendications précédentes **caractérisé en ce que** le détergent polyoxyéthylène est du BRIJ.

**7.** Procédé selon l'une quelconque des revendications précédentes **caractérisé en ce que** la concentration finale en détergent non ionique est comprise entre 0,025% et 0,05% en l'absence de saponine.

**8.** Procédé selon l'une quelconque des revendications précédentes **caractérisé en ce que** la concentration finale en saponine est comprise entre 0,10% et 4% en l'absence de détergent non ionique.

**9.** Procédé selon l'une quelconque des revendications précédentes **caractérisé en ce que** la concentration finale en endonucléase est supérieure à 0,1%.

**10.** Procédé selon l'une quelconque des revendications précédentes **caractérisé en ce que** l'endonucléase est mise en contact avec l'échantillon avant l'endopeptidase ou après inactivation de l'endopeptidase.

**11.** Procédé selon l'une quelconque des revendications précédentes **caractérisé en ce que** la concentration finale en endopeptidase est en excès.

**12.** Procédé selon l'une quelconque des revendications précédentes **caractérisé en ce que** la membrane poreuse a des pores compris entre 0,32 et 0,45 µm.

**13.** Procédé selon l'une quelconque des revendications précédentes **caractérisé en ce que** l'échantillon est frais.

**14.** Procédé pour la détection de microorganismes potentiellement présents dans un échantillon comprenant 10⁶ à 10⁸ cellules immunitaires ou cellules progénitrices de ces dernières, comprenant les étapes suivantes :
- Effectuer le procédé d'isolement selon l'une quelconque des revendications 1 à 13
- Effectuer un marquage des microorganismes viables retenus sur la membrane poreuse avec un marqueur de viabilité
- Effectuer un balayage par un faisceau laser ou par caméra de la membrane poreuse
- Déterminer la présence des microorganismes viables capturés par la membrane

**16.** Kit de lyse comprenant :
- un tampon de lyse comprenant un détergent non-ionique à une concentration comprise entre 0,004% et 0,050%
- une solution comprenant une saponine à une concentration comprise entre 0,03% et 4%
- une endonucléase
- une endopeptidase agissant à un pH compris entre 7 et 8.
